# NEUE EUROPÄISCHE PATENTSCHRIFT

(11) **EP 1 260 202 B2**
(45) Veröffentlichungstag und Bekanntmachung des Hinweises auf die Entscheidung über den Einspruch: **08.12.2010**
(45) Hinweis auf die Patenterteilung: 24.08.2005
(21) Anmeldenummer: 02005281.7
(22) Anmeldetag: 11.03.2002
(51) Int. Cl.: A61F 9/01, A61F 9/008

(54) **Lasersystem für die Hornhauttransplantation**
Laser system for corneal transplant surgery
Système à laser pour la transplantation cornéenne

(30) Priorität: 18.05.2001 DE 10124358
(43) Veröffentlichungstag der Anmeldung: 27.11.2002
(73) Patentinhaber: WaveLight Laser Technologie AG, 91058 Erlangen (DE)
(72) Erfinder: Donitzky, Christof, 90542 Eckental (DE)
(74) Vertreter: von Hellfeld, Axel

(56) Entgegenhaltungen:
- EP-A- 1 138 291
- WO-A-93/08877
- WO-A-94/09849
- US-A- 4 907 586
- US-A- 5 984 916
- US-A- 5 993 438

## Beschreibung

Die Erfindung betrifft ein Computerprogrammprodukt zum Steuern eines Lasersystems für die Hornhauttransplantation und ein derartiges Lasersystem.

Eine Transplantation eines Teils der menschlichen Hornhaut (Kornea) kann aus unterschiedlichen Ursachen indiziert sein. Z.B. kommt es beim sogenannten Keratokonus zu einer irregulären Veränderung der Hornhautform, so dass die optische Abbildung zu stark leidet. Auch bei extremer Eintrübung der Kornea durch z.B. Endothelzellverlust, Infektionen, Geschwüre, erbliche Erkrankungen oder Vernarbung kann eine Hornhauttransplantation notwendig machen.

Bei einer Hornhauttransplantation wird der zentrale Teil der erkrankten Hornhaut im Bereich der optischen Achse chirurgisch entnommen und es wird ein gesundes Spendertransplantat eingesetzt.

Im Stand der Technik erfolgt die Entnahme der Empfänger- als auch der Spenderhornhaut durch eine sogenannte Trepanation. Hierfür werden im Stand der Technik mechanische Instrumente eingesetzt, insbesondere ein sogenannter Hand- oder Motortrepan. Solche Trepane gemäß dem Stand der Technik ermöglichen eine Entnahme von scheibenförmigen Korneaabschnitten. Die bekannten Trepane weisen weisen einen kreiszylinderförmigen Hohlkörper auf, der am unteren Ende zu einer scharfen Schneidfläche geschliffen ist. Der Durchmesser der kreisförmigen Schliffkante entspricht etwa dem Durchmesser des herauszuschneidenden zentralen Hornhautabschnittes. Dabei ist sorgfältig darauf zu achten, dass das Endothel geringstmöglich traumatisiert wird. Ein weiterer wichtiger Gesichtspunkt bei der Hornhauttransplantation ist ein wasserdichter Wundverschluss.

Die mechanische Hornhautchirurgie mit einem Trepan hat zunächst den Nachteil, dass die geometrische Struktur des herausgeschnittenen Hornhautabschnittes festgelegt ist aufgrund der vorgegebenen Schliffkante; es sind nur kreiszylindrische Abschnitte anschneidbar aufgrund der kreiszylinderförmigen Gestalt des Trepans. Auch bedingt die Tendenz zur Schnittablenkung im Lammellengewebe in Abhängigkeit von der Wölbung der Hornhaut Probleme hinsichtlich der genauen Reproduzierbarkeit des Schnittes. Ist ein Trepan einmal angesetzt, kann nur noch die Tiefe des Schnittes gesteuert werden. Hiefür kennt der Stand der Technik beim Trepanschnitt unterschiedliche Verfahren, z.B. den Einsatz von Stempeln im Trepan.

Ein Hauptproblem bei der Hornhauttransplantation ist der flüssigkeitsdichte Wundverschluss. Im Stand der Technik wird das Hornhauttransplantat nach erfolgreicher Implantation durch das Legen einer Naht fixiert. Die Naht verbleibt typischerweise für einen Zeitraum von bis zu einem Jahr in der Empfängerhornhaut. Eine solche Naht ist nicht nur sehr aufwendig, sondern kann auch zu diversen Komplikationen führen, insbesondere kann sie eine fehlerhafte optische Position des Spendertransplantates bewirken oder auch zu einer unzureichenden Flüssigkeitsabdichtung. Auch kann die Naht die optischen Eigenschaften des Abbildungssystems "Auge" verändern, z.B. können durch die Naht Verzerrungen in der Hornhaut induziert werden, die zu einem Astigmatismus führen können.

Die Flüssigkeitsabdichtung ist ein grundlegendes Problem. Das menschliche Auge hat im Augapfel einen relativen Überdruck von im Normalfall etwa 15mmHg.

Das Dokument US-A-5984916 beschreibt ein ophtalmologisches Lasersystem, bei dem Teile der Hornhaut entfernt werden, jedoch die inneren Abschnitte der Hornhaut unversehrt bleiben, sodass ein flüssigkeitsdichter Wundverschluss und eine stabile Positionierung eines Transplantates dort nicht das Hauptproblem sind.

Der Erfindung liegt die Aufgabe zugrunde, ein Lasersystem und ein Computerprogrammprodukt zum Steuern desselben für eine Hornhauttransplantation bereitzustellen, mit denen bei einem vollständigen axialen Herausschneiden eines Abschnittes der Hornhaut gleichwohl ein flüssigkeitsdichter Wundverschluss und eine genaue Positionierung eines Transplantates ermöglicht sind.

Ein hierfür geeignetes Computerprogrammprodukt zum Steuern eines Lasersystems ist im Patentanspruch 1 beschrieben, während ein Lasersystem zur Lösung der genannten Aufgabe im Anspruch 4 beschrieben ist. Bevorzugte Ausgestaltungen der Erfindung sind in den abhängigen Ansprüchen beschrieben.

Ein derartiges Lasersystem ermöglicht eine Hornhauttransplantation mit sogenannten photodisruptivem Laserschneiden.

Die Erzeugung extrem kurzer Laserpulse hat es in jüngster Zeit insbesondere bei der ophtalmologischen Chirurgie ermöglicht, durch Fokussierung der Laserpulse in Inneren der Hornhaut z.B. im Rahmen der LASIK-Behandlung ohne mechanische Schnittmittel ein Hornhautläppchen abzutrennen. Kurze Laserpulse in Nanosekundenbis Femtosekundenbereich haben so hohe Leistungen, dass mit einer geeigneten Fokussierung das biologische Material in seinem Inneren "geschnitten" werden kann, ohne dass es zu inneren Wärmeeffekten oder dergleichen kommt.

Durch die erfindungsgemäß erreichte Hinterschneidung im "Schnitt" der Hornhaut wird eines Selbstabdichtung erreicht, das der oben angesprochene relative Überdruck im Auge ein sattes Anliegen des Implantates an der Rest-Hornhaut des Empfängers bewirkt. Weiterhin hat die erfindungsgemäß erreichte Schnittform den Vorteil, dass sie auch die optische Zentrierung des Spenderimplantates in der Empfängerhornhaut fördert. Auch wird hierdurch die Vernähung des eingesetzten Implantates auf ein Minimum reduziert und es werden die dadurch bedingten Nachteile weitestgehend vermieden.

Gemäß einer bevorzugten Ausgestaltung der Erfindung ist die Hinterschneidung zick-zack-förmig. Die Hinterschneidung ist so angelegt, dass, aus dem Inneren des Auges gesehen, ein Teil der verbliebenen Empfänger-Hornhaut das eingesetzte Spender-Implantat außenseitig in Bezug auf die optische Achse radial nach innen übergreift. Eine besonders bevorzugte Ausgestaltung des Steuerprogramms der Rechnereinheit für das Lasersystem sieht vor, daß die Fokussteuerung eine Hinterschneidung in der Hornhaut bewirkt, die einen geschnittenen Abschnitt erzeugt, der sich radial in bezug auf die Achse der Kornea erstreckt. Dieser Radialabschnitt erzeugt eine Dichtfläche, die aufgrund des Binnendruckes im Auge optimal als ringförmige Dichtfläche wirkt. Die plan-parallelen Flächen des Implantates einerseits und der Rest-Hornhaut andererseits liegen satt und großflächig unter dem Überdruck des Auges aneinander und die durch die Druckdifferenz bewirkte Kraft steht im wesentlichen senkrecht auf diesen Dichtflächen.

Nachfolgend wird die Erfindung anhand der Zeichnung näher beschrieben. Es zeigt:
Figur 1 schematisch ein Lasersystem für die Hornhauttransplantation;
Figur 2 eine Draufsicht in axialer Richtung auf die Hornhaut mit der durch den bewegten Laserfokus erzeugten Bahn;
Figur 3 einen schematischen Axialschnitt durch die verbliebene Empfängerhornhaut und das eingesetzte Spenderimplantat; und
Figur 4 ein anderes Ausführungsbeispiel für eine Schnittform, die in Abwandlung der Schnittform von Figur 3 mit einem erfindungsgemäßen Lasersystem erzielbar ist.

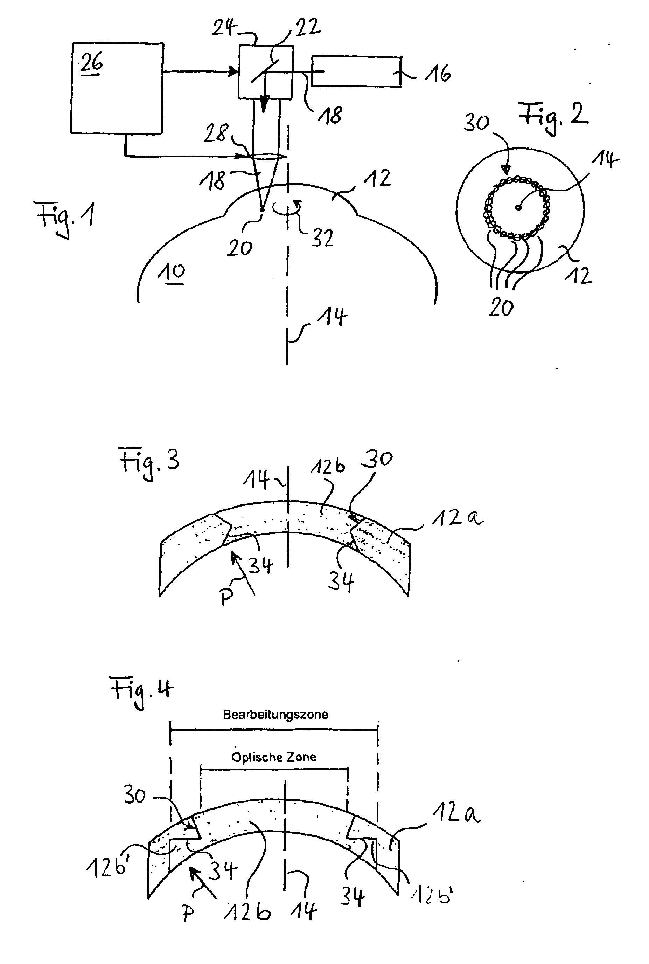

Figur 1 zeigt schematisch ein Auge 10 und dessen Kornea 12. Dabei kann es sich sowohl um ein Empfängerauge, aus dem ein zentraler Kornea-Abschnitt herausgeschnitten werden soll, als auch um ein Spender-Auge handeln, aus dem ein zentrales Implantat zum Einsetzen in das Empfängerauge herausgeschnitten werden soll. Die nachfolgend beschriebenen Schnitte sind komplementär, d. h. der Schnitt im Empfängerauge zum Entfernen des erkrankten oder irregulären Korneaabschnittes entspricht dem Schnitt, mit dem aus der Spenderkornea das Implantat entnommen wird.

Der Schnitt erfolgt durch photodisruptives Laserschneiden, d. h. ein Laserstrahl wird so im Inneren des Gewebes fokussiert, daß er dort aufgrund seiner hohen momentanen Leistung eine Materialauflösung (Ablation) bewirkt und der Fokus wird dann sequentiell entlang einer Bahn bewegt, so daß insgesamt ein Schnitt entsteht.

Eine Laserstrahlquelle 16 emittiert Laserstrahlpulse 18. Gute Ergebnisse werden erzielt mit Wellenlängen im nahen Infrarotbereich, in dem die Kornea eine hohe Durchlässigkeit hat. Die bevorzugten Laserpulslängen liegen im Bereich von 1fs bis 10ns und die Pulsenergien im Bereich von 1nJ-5mJ.

Mittels optischer Ablenk- und Fokussiereinrichtungen wird der Laserstrahl 18 so fokussiert, daß der Fokus 20 an der Stelle positioniert ist, an der die Photodisruption erfolgen soll, also insbesondere auch im Inneren der Hornhaut, wie in Figur 1 schematisch dargestellt ist. Die optischen Mittel zum Steuern der Position der Laserpulse und zum Steuern der Position des Fokus sind in Figur 1 schematisch durch einen Spiegel 22 und eine Umlenkeinheit 24 repräsentiert sowie durch eine Linseneinheit 28, die die Fokussierung bewirkt. Die dargestellten Elemente, also insbesondere der Spiegel 22 und die Fokussiereinheit 28 sind so bewegbar, daß die nachfolgend beschriebene Steuerung der Bahn des Fokus ermöglicht ist. Die in Rede stehenden optischen Mittel sind als solche im Stand der Technik bekannt und brauchen hier nicht näher beschrieben zu werden. Wesentlich ist die Steuerung der optischen Mittel entsprechend einem Rechnerprogramm, das in einer Rechnereinheit 26 abgelegt ist und den Rechner steuert, der seinerseits entsprechend dem Programm die optischen Mittel 22, 24, 28 zur Erzeugung der gewünschten Fokusbahn und damit der gewünschten Schnittform ansteuert.

Figur 2 zeigt schematisch eine Draufsicht in Richtung der Achse 14 der Kornea 12 und die durch die Steuerung der optischen Mittel erreichte Bahn 30 der Fokusse 20 (foci) der Laserpulse 18. Die Bahn der Fokusse ist rotationssymmetrisch um die optische Achse 14 der Kornea 12. Die Laserpulse werden also z. B. in Richtung des Pfeiles 32 um die optische Achse 14 herumgeführt und es entsteht so eine geschlossene Umlaufbahn um die Achse. In der Tiefe, d. h. in axialer Richtung, erfolgt die Steuerung der Positionen des Fokus entsprechend den Ausführungsbeispielen nach den Figuren 3 und 4 so, daß eine Hinterschneidung entsteht.

Figur 3, die keine erfindungsgemäße Ausführung zeigt, zeigt das verbliebene Teil 12a der Empfängerhornhaut und das Implantatteil 12b der Spenderhornhaut im eingesetzten Zustand, in dem der Verheilungsprozeß stattfinden soll. Somit wurde beim Herausschneiden des Spender-Hornhautabschnittes 12b das Lasersystem gemäß Figur 1 so gesteuert, daß die in Figur 3 gezeigte Struktur entstanden ist. Die Schnittbahn ist mit dem Bezugszeichen 30 gekennzeichnet. Entsprechend wurde auch im Empfängerauge ein zentraler Abschnitt entfernt, dessen Form dem Abschnitt 12b entspricht.

Die Bahn 30 der Fokusse, d. h. die Schnittlinie beim photodisruptiven Laserschneiden, ist beim Ausführungsbeispiel gemäß Figur 3 in der dargestellten Weise abgeknickt, also zick-zack-förmig in dem Sinne, daß eine Hinterschneidung gegeben ist.

Die dargestellte Schnittführung erreicht eine exakte Zentrierung des Spenderhornhautabschnittes 12b in der verbliebenen Empfängerhornhaut 12a. Es erfolgt eine Selbstdichtung aufgrund des Innendruckes P im Auge. Die Dichtfläche ist der in Figur 3 mit dem Bezugszeichen 34 versehene innere Abschnitt des Schnittes 30, also die sog. Hinterschneidung. Beim Ausführungsbeispiel gemäß Figur 3 wird in der Regel eine Fixierung des Implantates 12b mittels einer kleinen Naht erforderlich sein. Allerdings kann diese Naht so wenig aufwendig durchgeführt werden, daß die oben skizzierten Probleme des Standes der Technik weitestgehend vermieden sind.

Figur 4 zeigt eine erfindungsgemäße Schnittführung, bei der eine Nahtfixierung noch weiter verringert werden kann. Die Bahn 30 der Laserfokusse ist gemäß Figur 4 so zick-zack-förmig, daß eine Hinterschneidung 34 entsteht, die sich radial in bezug auf die optische Achse 14 der Kornea erstreckt. Hierdurch wird eine relativ große Dichtfläche erreicht. Die entstehenden Anlageflächen zwischen Implantat 12b und Resthornhaut 12a sind so, daß die Hinterschneidung 34 eine optimale Dichtwirkung und Positionierung des Implantates bewirkt, im Wechselspiel mit dem Überdruck P, dessen Richtung in Figur 3 und 4 durch den Pfeil angedeutet ist. Die in bezug auf die optische Achse 14 der Kornea radiale Hinterschneidung 34 bedeutet, daß das Implantat 12b in der Resthornhaut 12a eine stabile Position einnimmt, d. h. der Druck P drückt den Abschnitt 12b stabil (und nicht labil) in eine komplementäre Ausnehmung in der Resthornhaut 12a.

Figur 4 zeigt auch die Flächenverhältnisse hinsichtlich der optischen Zone und der sog. Bearbeitungszone. Die geschnittene Bahn 30 ist so angelegt, daß die Flächendifferenz zwischen der gezeigten Bearbeitungszone und der gezeigten optischen Zone größer ist als die optische Zone selbst. Bei diesem erfindungsgemäßen Ausführungsbeispiel ergibt sich eine ringförmige Dichtfläche (in der Bearbeitungszone außerhalb der optischen Zone) mit einer Anpreßkraft, insbesondere im Bereich der Hinterschneidung 34, die größer ist als die Kraft, die aufgrund des Binnendrukkes P in der optischen Zone wirksam ist. Ein Beispiel: Die optische Zone habe einen Durchmesser von 7 mm, also eine Fläche von etwa 38,5 mm². Die Bearbeitungszone habe einen Durchmesser von 10 mm, also eine Fläche von etwa 78,5 mm². Dies ergibt eine Dichtfläche im Bereich der Hinterschneidung 34 von etwa 40 mm², also mehr als die Fläche der optischen Zone. Allgemein läßt sich ableiten, daß es vorteilhaft ist, wenn die im vorstehenden Sinne definierte Dichtfläche (Differenz zwischen Bearbeitungszone und optischer Zone gemäß Figur 4) größer ist als die optische Zone oder zumindest 75 % davon beträgt. Eine Obergrenze für die Dichtfläche ist durch die Geometrie des Auges selbst gegeben und durch eine gemäß den vorstehenden Kriterien sinnvolle Schnittführung, so daß sich eine Zahlenangabe hierzu erübrigt.

Die Schnittführung gemäß Figur 4 erlaubt eine minimalste Nahtlegung, die nur soweit erforderlich ist, daß ein Verdrehen oder Luxieren des Implantates verhindert ist.

## Patentansprüche

1. Computerprogrammprodukt zur Steuerung eines Lasersystems mit einer Laserstrahlquelle (16), die Laserpulse (18) mit Wellenlängen emittiert, für die die menschliche Kornea (12) transparent ist, und mit Pulslängen im Nano- bis Femto-Sekundenbereich, und mit optischen Mitteln (22, 24, 28), die von dem Computer so gesteuert werden, dass der Fokus (20) der Laserpulse (18) eine Bahn (30) durch die Kornea durchläuft, **dadurch gekennzeichnet, dass** das Computerprogrammprodukt Programmabschnitte aufweist, die bewirken, dass mittels der Bahn (30) der Fokusse eine zentraler Abschnitt (12b) der Hornhaut vollständig axial aus der Hornhaut (12a) herausgeschnitten wird, wobei die Bahn (30) in axialer Richtung zumindest eine Hinterschneidung aufweist mit einer Dichtfläche (34), die zumindest 75% der Fläche der optischen Zone der Kornea entspricht und die sich radial in Bezug auf die optische Achse (14) der Kornea erstreckt.

2. Computerprogrammprodukt nach Anspruch 1, **dadurch gekennzeichnet, dass** die Dichtfläche (34) größer ist als die Fläche der optischen Zone der Kornea.

3. Computerprogrammprodukt nach einem der Ansprüche 1 oder 2, **dadurch gekennzeichnet, dass** die Bahn (30) zick-zack-förmig ist.

4. Lasersystem mit
- einer Laserstrahlquelle (16), die Laserpulse (18) mit Wellenlängen emittiert, für die die menschliche Kornea (12) transparent ist, und mit Pulslängen im Nano- bis Femto-Sekundenbereich , und
- optischen Mitteln (22, 24, 28) zum Steuern der Position und des Fokus (20) der Laserpulse (18), **gekennzeichnet durch**
- eine derart programmgesteuerte Rechnereinheit (26), dass sie die optischen Mittel (22, 24, 28) so steuert, dass die Fokusse (20), bezogen auf eine optische Achse (14) der Kornea (12), eine um eine Achse (14) geführte Bahn (30) im Inneren der Kornea beschreiben, **durch** die ein zentraler Abschnitt (12b) der Hornhaut vollständig axial aus der Hornhaut (12a) herausgeschnitten wird und die in axialer Richtung zumindest eine Hinterschneidung aufweist mit einer Dichtfläche (34), die mindestens 75% der Fläche der optischen Zone der Kornea entspricht und die sich radial in Bezug auf die optische Achse (14) der Kornea erstreckt.

5. Lasersystem nach Anspruch 4, **dadurch gekennzeichnet, dass** die Dichtfläche (34) größer ist als die Flache der optischen Zone der Kornea.

6. Lasersystem nach einem der Ansprüche 4 oder 5, **dadurch gekennzeichnet, dass** die Bahn (30) zick-zack-förmig ist.

## Claims

1. Computer program product for controlling a laser system comprising a laser-beam source (16) emitting laser pulses (18) with wavelengths in respect of which the human cornea (12) is transparent, and with pulse lengths in the nanosecond to femtosecond range, and comprising optical means (22, 24, 28) which are controlled by the computer in such a way that the focus (20) of the laser pulses (18) follows a path (30) through the cornea, **characterized in that** the computer program product has program sections whose effect is such that a central portion (12b) of the cornea is cut completely axially from the cornea (12a) by means of the path (30) of the foci, the path (30) having at least one undercut, in the axial direction, with a sealing surface (34) which corresponds to at least 75% of the surface area of the optical zone of the cornea and which extends radially in relation to the optical axis (14) of the cornea.

2. Computer program product according to Claim 1, **characterized in that** the sealing surface (34) is larger than the surface area of the optical zone of the cornea.

3. Computer program product according to either of Claims 1 and 2, **characterized in that** the path (30) is zigzag-shaped.

4. Laser system comprising
- a laser-beam source (16) emitting laser pulses (18) with wavelengths in respect of which the human cornea (12) is transparent, and with pulse lengths in the nanosecond to femtosecond range, and
- optical means (22, 24, 28) for controlling the position and the focus (20) of the laser pulses (18), **characterized by**
- a computer unit (26) which is programmed in such a way that it controls the optical means (22, 24, 28) such that, in relation to an optical axis (14) of the cornea (12), the foci (20) describe a path (30), in the interior of the cornea, which is guided about an axis (14) and by which a central portion (12b) of the cornea is cut completely axially from the cornea (12a), and which path (30) has at least one undercut, in the axial direction, with a sealing surface (34) which corresponds to at least 75% of the surface area of the optical zone of the cornea and which extends radially in relation to the optical axis (14) of the cornea.

5. Laser system according to Claim 4, **characterized in that** the sealing surface (34) is larger than the surface area of the optical zone of the cornea.

6. Laser system according to either of Claims 4 and 5, **characterized in that** the path (30) is zigzag-shaped.

## Revendications

1. Programme informatique pour commander un système laser comprenant une source de rayonnement laser (16) qui émet des impulsions laser (18) avec des longueurs d'onde pour lesquelles la cornée humaine est transparente (12) et des longueurs d'impulsion dans la plage des nano aux femtosecondes, et des moyens optiques (22, 24, 28) qui sont commandés par l'ordinateur de telle sorte que le foyer (20) des impulsions laser (18) parcourt un trajet (30) à travers la cornée, **caractérisé en ce que** le programme informatique présente des segments de programme qui provoquent qu'au moyen du trajet (30) des foyers un segment central (12b) de la cornée est découpé entièrement axialement de la cornée (12a), le trajet (30) présentant dans la direction axiale au moins une contre-dépouille avec une surface d'étanchéité (34) qui correspond à au moins 75 % de la surface de la zone optique de la cornée et qui s'étend radialement par rapport à l'axe optique (14) de la cornée.

2. Programme informatique selon la revendication 1, **caractérisé en ce que** la surface d'étanchéité (34) est plus grande que la surface de la zone optique de la cornée.

3. Programme informatique selon l'une quelconque des revendications 1 ou 2, **caractérisé en ce que** le trajet (30) est en zigzag.

4. Système laser comprenant
■ une source de rayonnement laser (16) qui émet des impulsions laser (18) avec des longueurs d'onde pour lesquelles la cornée humaine est transparente (12) et des longueurs d'impulsion dans la plage des nano aux femtosecondes, et
■ des moyens optiques (22, 24, 28) pour commander la position et le foyer (20) des impulsions laser (18), **caractérisé par**
■ une unité de calcul (26) commandée par un programme de manière à ce qu'elle commande les moyens optiques (22, 24, 28) de telle sorte que les foyers (20), par rapport à un axe optique (14) de la cornée (12), décrivent à l'intérieur de la cornée un trajet (30) guidé autour d'un axe (14) par lequel un segment central (12b) de la cornée est découpé entièrement axialement de la cornée (12a) et qui présente dans la direction axiale au moins une contre-dépouille avec une surface d'étanchéité (34) qui correspond à au moins 75 % de la surface de la zone optique de la cornée et qui s'étend radialement par rapport à l'axe optique (14) de la cornée.

5. Système laser selon la revendication 4, **caractérisé en ce que** la surface d'étanchéité (34) est plus grande que la surface de la zone optique de la cornée.

6. Système laser selon l'une quelconque des revendications 4 ou 5, **caractérisé en ce que** le trajet (30) est en zigzag.
